# FASCICULE DE BREVET EUROPEEN

(11) **EP 1 361 836 B1**
(45) Date de publication et mention de la délivrance du brevet: **16.01.2008**
(21) Numéro de dépôt: 02704878.4
(22) Date de dépôt: 22.02.2002
(51) Int. Cl.: A61F 2/16

(54) **IMPLANT INTRAOCULAIRE EN MATERIAU SOUPLE**
INTRAOKULARES IMPLANTAT AUS FLEXIBLEM MATERIAL
FLEXIBLE INTRAOCULAR IMPLANT

(30) Priorité: 23.02.2001 FR 0102466
(43) Date de publication de la demande: 19.11.2003
(73) Titulaire: CORNEAL INDUSTRIE, 74370 Pringy (FR)
(72) Inventeur: VINCHON, Cyrille, F-74370 Chilly (FR); BOS, Gilles, F-74000 Annecy (FR)
(74) Mandataire: Dronne, Guy
(86) Numéro de dépôt international: PCT/FR2002/000657
(87) Numéro de publication internationale: WO 2002/067818

(56) Documents cités:
- EP-A- 1 108 402
- WO-A-97/12564
- US-A- 5 928 282

## Description

La présente invention a pour objet un implant intraoculaire réalisé en un matériau souple et notamment un implant de chambre antérieure du type réfractif.

Il est bien connu de réaliser maintenant l'intégralité d'un implant intraoculaire, c'est-à-dire à la fois sa partie optique et sa partie haptique, en un matériau souple du type acrylique hydrophile formant un hydrogel tel que le matériau commercialisé sous la marque Hydrogel ou en gel de silicone. En effet, lorsque ces implants sont réalisés en matériau souple, il est possible de plier ou d'enrouler la partie optique et la partie haptique autour d'un diamètre de la partie optique pour en réduire les dimensions externes et diminuer ainsi la dimension de l'incision qu'il est nécessaire de réaliser dans la cornée de l'oeil pour la mise en place de l'implant. On sait en effet que la diminution de l'incision à réaliser favorise considérablement la cicatrisation de la cornée après l'intervention, permet d'éviter l'apparition d'astygmatisme post-opératoire induit et rend donc l'intervention chirurgicale en elle-même beaucoup plus légère.

On sait également que, pour la mise en place de l'implant dans l'oeil, on a conçu des dispositifs chirurgicaux appelés "injecteurs" qui permettent, dans un premier temps, de procéder automatiquement au pliage ou enroulement de l'implant puis, dans un deuxième temps, à l'aide d'une canule introduite dans l'oeil, de pousser à l'aide d'un piston l'implant plié dans la canule afin de permettre la mise en place de l'implant dans la partie souhaitée de l'oeil.

On sait également que le problème spécifique soulevé par la réalisation d'implants intraoculaires entièrement fabriqués en matériau souple est que les propriétés mécaniques de ces matériaux sont bien sûr très différentes des propriétés mécaniques du matériau antérieurement utilisé qui était le PMMA. En particulier, lorsque l'implant est mis en place dans l'oeil, quelle que soit la zone où il doit être mis, c'est-à-dire dans la chambre antérieure, comme c'est le cas de préférence pour la présente invention, dans la chambre postérieure ou dans le sac capsulaire, les parties haptiques de l'implant tendent sous l'effet des contraintes mécaniques qui lui sont appliquées à provoquer un déplacement vers l'avant de la partie optique, ce phénomène étant le plus souvent appelé "projection". Cette projection vers l'avant peut modifier l'effet correcteur de l'implant à l'intérieur de l'oeil ou peut entraîner des lésions de certaines parties de la paroi interne de l'oeil, et notamment de l'endothélium cornéen qui est particulièrement sensible.

Il est également important de permettre l'immobilisation de l'implant à l'intérieur de l'oeil, c'est-à-dire en particulier d'éviter sa rotation autour de l'axe optique de l'oeil. Cela est particulièrement vrai dans le cas où la partie optique présente des propriétés de correction optique qui ne sont pas de révolution autour de son axe optique. Il s'agit également de supprimer les phénomènes d'inflammation et d'endommagement des tissus générés par la rotation.

Un implant intraoculaire monobloc selon le preambule de la revendication 1 est décrit dans le document US-A-5 928 282.

Un objet de la présente invention est de fournir un implant intraoculaire souple monobloc qui permette, après la mise en place de l'implant dans l'oeil et notamment dans" la chambre antérieure, d'éviter le phénomène de projection vers l'avant de l'implant.

Pour atteindre ce but selon l'invention, l'implant intraoculaire monobloc, réalisé en un matériau souple comprenant une partie optique sensiblement circulaire d'axe optique XX' et de centre O et une partie haptique constituée par deux ensembles haptiques sensiblement diamétralement opposés, se caractérise en ce que chaque ensemble haptique comprend
- un bras radial s'étendant radialement par rapport à la partie optique (10) et ayant une première extrémité de raccordement à la partie optique et une deuxième extrémité
- deux éléments d'appui massifs et sensiblement identiques, chaque élément d'appui présentant un bord d'appui sur la paroi interne de l'oeil ; et
- deux bras de flexion sensiblement identiques, chaque bras de flexion ayant une première extrémité raccordée à la première extrémité desdits bras de flexion et une deuxième extrémité raccordée à un élément d'appui en dehors de son bord d'appui, chaque bras de flexion ayant sur au moins une partie de sa longueur une épaisseur (w) selon la direction de l'axe optique (XX') supérieure à sa largeur (h) dans un plan orthogonal à l'axe optique.

On comprend que les deux éléments d'appui de chaque ensemble haptique sont relativement massifs et sont reliés mécaniquement à la partie optique par des bras de flexion qui ont une forme telle qu'ils permettent une flexion uniquement autour d'un axe parallèle à l'axe optique de l'implant. On comprend qu'ainsi, lorsque des contraintes appliquées à la partie haptique résultant de la mise en place de l'implant dans l'oeil, les éléments d'appui des ensembles haptiques soumis aux contraintes vont entraîner une flexion des bras de flexion autour d'axes parallèles à l'axe optique de l'Implant, cette déformation résultant des contraintes appliquées n'entraînera pas de projection ou n'entraînera pas de projection significative puisque la déformation reste dans un plan orthogonal à l'axe optique.

On comprend en outre que la présence des bras radiaux qui sont de préférence massifs ne modifie pas le mode de déformation de la partie haptique sous l'effet des contraintes, mais augmente la stabilité de l'ensemble de l'implant sous l'effet des contraintes qui lui sont appliquées.

De préférence également, la face postérieure de chaque ensemble haptique comporte une portion allongée en saillie s'étendant sur la deuxième extrémité du bras radial, sur les deux bras de flexion et sur une partie des éléments d'appui, par quoi ladite portion allongée en saillie (28) rigidifie l'ensemble haptique dans le plan orthogonal à l'axe optique X, X'.

Cette portion faisant saillie dans la face postérieure de la partie haptique et s'étendant au niveau de l'extrémité des bras radiaux, au niveau des bras de flexion et au niveau des éléments d'appui, constitue un élément de renfort mécanique ou de rigidification vis-à-vis de déformations dans des directions non contenues dans le plan orthogonal à l'axe optique. Cette portion en saillie, en forme générale d'arc de cercle, permet donc de limiter encore les risques de "projection" de l'Implant.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées, sur lesquelles :
- la figure 1A est une vue de face d'un premier mode de réalisation de l'implant ;
- la figure 1B est une vue de côté de l'implant représenté sur la figure 1A ;
- la figure 2 est une vue de détail montrant la coopération entre un ensemble haptique et la partie interne de l'oeil ;
- la figure 3 est une vue partielle de la figure 1A montrant plus en détail notamment un mode de réalisation des bras de flexion ; et
- la figure 4 est une vue en coupe selon la ligne IV-IV de la figure 3.

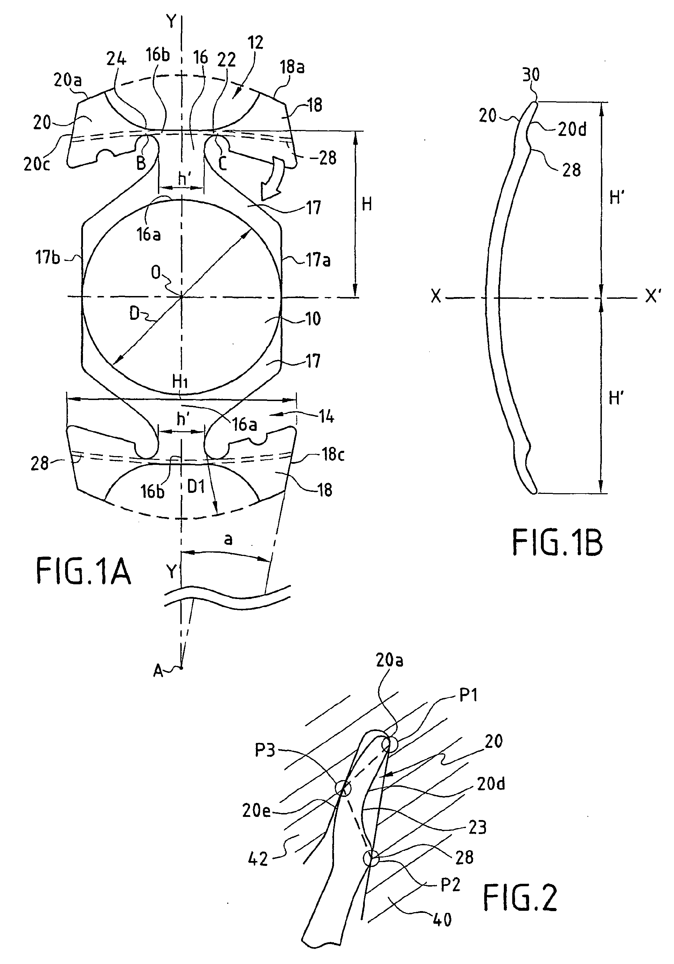

En se référant tout d'abord aux figures 1 A et 1B, on va décrire les éléments essentiels de l'implant souple.

Comme cela est bien connu, l'implant est constitué par une partie optique 10 de forme sensiblement circulaire, de diamètre D, de centre O et d'axe optique XX'. Cette partie optique présente une périphérie référencée 10a.

L'implant comporte également deux ensemble haptiques 12 et 14 qui sont disposés symétriquement par rapport à l'axe optique XX'. Ces deux ensembles haptiques étant sensiblement identiques, on ne décrira en détail que l'ensemble haptique 12. Dans le mode de réalisation considéré, chaque ensemble haptique comporte un bras radial 16 dont une première extrémité 16a est raccordée à la périphérie de la partie optique 10 et dont l'autre extrémité 16b est libre et se trouve à une distance H du centre O de la partie optique. Dans le mode de réalisation considéré, la première, extrémité 16a du bras radial 16 est "noyée" dans une extension 17 de la périphérie de la partie optique. Cette extension 17, pour chaque ensemble haptique, est limitée par deux bords 17a et 17b parallèles à l'axe principal de l'implant YY'. La distance entre les bords 17a de l'extension 17 est égale au diamètre D de la partie optique.

L'ensemble haptique 12 comporte également deux éléments d'appui massifs 18 et 20 symétriques par rapport à l'axe longitudinal YY' de l'implant. Chaque élément d'appui comporte un bord d'appui 18a, 20a destiné à venir au contact, ainsi qu'on l'expliquera ultérieurement, de la paroi interne de l'oeil. La géométrie de ces bords en contact assurent de faibles pressions sur les tissus environnants, notamment sur l'iris et sur le canal de Schlemm qui assure le drainage de la chambre antérieure. Les bords d'appui 18a, 20a sont sensiblement disposés sur un cercle de centre O et de rayon D1. Chaque élément d'appui 18, 20 est raccordé à la deuxième extrémité 16b du bras radial 16 par des bras de flexion respectivement référencés 22 et 24.

Comme on l'expliquera ultérieurement plus en détail, les bras de flexion 22 et 24 de chaque ensemble haptique définissent des axes de pivotement repérés par les lettres B et C, ces axes étant parallèles à l'axe optique XX'. On comprend qu'ainsi, lorsque l'implant est mis dans l'oeil, les contraintes mécaniques dues au fait que, au repos, le diamètre D1 est supérieur au diamètre interne de l'oeil entraînent un mouvement de flexion des bras de flexion 22 et 24 produisant une rotation des éléments d'appui 18 et 20, ces éléments d'appui restant dans le plan orthogonal à l'axe optique XX' et évitant ainsi tout effet de projection.

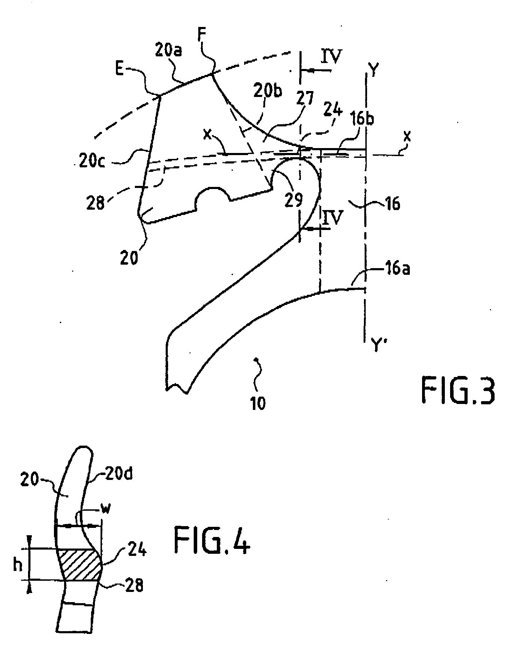

En se référant maintenant plus particulièrement aux figures 3 et 4, on va décrire plus en détail un mode de réalisation préféré des bras de flexion et des éléments d'appui.

Si l'on considère la face postérieure de l'ensemble haptique 12, celle-ci comporte une portion en saillie 28 qui a la forme générale d'un arc de cercle et qui s'étend sur la face postérieure 20d et 18d des éléments d'appui, sur la face postérieure des bras de flexion 22 et 24 et sur la face postérieure de l'extrémité 16b du bras radial. Comme cela est mieux visible sur la figure 4, la largeur h de la partie formant saillie 28 est très inférieure à l'épaisseur w (selon la direction de l'axe optique X, X') de la partie haptique au niveau de la saillie 28. De préférence, le rapport entre w et h est au moins égal à 2. La distance h définit ainsi également la largeur du bras de flexion au sens strict du terme, c'est-à-dire dans la région où il est le plus étroit et où il définit les axes de pivotement déjà mentionnés B et C.

On comprend également que cette portion en saillie 28 constitue "un cintre de flexion" formant une nervure de rigidification de cette partie de la portion haptique empêchant ainsi la déformation de la partie haptique en dehors du plan orthogonal à l'axe haptique XX'. En revanche, ce "cintre de flexion" permet la flexion des bras 22 et 24 autour des axes parallèles à XX' et passant par les points B et C.

De plus, les bras radiaux 16 sont relativement massifs par rapport aux bras de flexion 22 et 24. Cela signifie que la largeur h' des bras radiaux est très supérieure à la largeur h des bras de flexion, alors que son épaisseur w' est égale à l'épaisseur courante des éléments d'appui 18 et 20. Par rapport aux bras de flexion 22 et 24, les bras radiaux 16 peuvent donc être considérés, en première approximation, comme non déformables sous l'effet des contraintes. Sous l'effet des contraintes résultant de la mise en place de l'implant dans l'oeil, ce sont donc uniquement les bras de flexion 22 et 24 qui vont fléchir.

Si l'on considère sur la figure 3 l'élément d'appui 20, on voit qu'il est limité par le bord d'appui 20a déjà décrit par un bord de raccordement 20b servant au raccordement avec l'extrémité du bras de flexion 24, ce raccordement étant complété par des congés 27, 29, et par un bord externe 20c opposé au bord de raccordement 20b. Il est important de noter que les raccordements E et F entre le bord d'appui 20a et le bord externe 20c et entre le bord d'appui 20a et le bord de raccordement 20b forment des angles relativement marqués dépourvus d'arrondis. Ces angles E et F forment des "crampons" d'ancrage dans la paroi interne de l'oeil pour éviter la rotation accidentelle de l'implant autour de son axe XX' sous l'effet de chocs ou sous l'effet de contraintes appliquées.

Il faut également relever que le bord externe 18c, 20c des éléments d'appui 22 et 20 font un angle a avec l'axe longitudinal YY' de l'implant. Le sommet de cet angle A est opposé au centre O de la partie optique par rapport à l'extrémité 16b du bras radial. Cette angulation du bord externe des éléments d'appui favorise la rotation des éléments d'appui autour des axes B et C, dans le sens des flèches F, lorsque l'implant est disposé dans l'injecteur en vue de son pliage. Pour favoriser cet effet, il est important de relever que la plus grande largeur H1 de chaque ensemble haptique 12 et 14 est supérieure au diamètre D de la partie optique. Cette disposition favorise le pivotement des éléments d'appui 18 et 20 lorsque l'implant est placé dans la partie de l'injecteur permettant d'obtenir son pliage. Les éléments de pliage n'entrent en contact avec la partie optique qu'ultérieurement puisque H1 est supérieure à D.

En se référant maintenant à la figure 2, on va décrire une autre caractéristique de l'implant intraoculaire selon un mode préféré de réalisation qui permet de favoriser la stabilité de l'implant mis en place dans l'oeil. Cette figure montre que la face postérieure 20d de l'élément d'appui 20 comporte une portion de surface concave 23 reliant la portion en saillie 28, déjà décrite, aux bords d'appui 18a, 20a. En revanche, la face antérieure 20e de l'élément d'appui 20 présente une forme convexe. Lorsque l'extrémité de la partie haptique est mise en place dans la chambre antérieure dans l'angle irido cornéen entre la face antérieure de l'iris 40 et la face postérieure 42 de la scière, c'est-à-dire dans le sulcus, l'élément d'appui est en contact avec ces différentes parties de la cavité interne de l'oeil en trois points, dans le plan de coupe de la figure 2, respectivement repérés par P₁, P₂ et P₃. En réalité, le contact se produit selon trois segments curvillignes. Ces trois contacts assurent une grande stabilité de l'appui de chaque élément d'appui, c'est-à-dire de chaque ensemble haptique sur la paroi interne de l'oeil.

De préférence, la distance (H) entre le centre (0) de la partie optique (10) et la deuxième extrémité (16b) d'un bras radial est inférieure à la demi-longueur hors tout (H') de l'ensemble de l'implant.

## Revendications

1. Implant intraoculaire monobloc réalisé en un matériau souple comprenant une partie optique (10) sensiblement circulaire à axe optique XX' et de centre 0, et une partie haptique constituée par deux ensembles haptiques (12, 14) sensiblement diamétralement opposés, chaque ensemble haptique comprend :
- deux éléments d'appui (18, 20) massifs et sensiblement identiques, chaque élément d'appui présentant un bord d'appui (18a, 20a) sur la paroi interne de l'oeil ; et
- deux bras de flexion sensiblement identiques (22, 24), chaque bras de flexion ayant une première extrémité et une deuxième extrémité raccordée à un élément d'appui (18, 20) en dehors de son bord d'appui (18a, 20a), chaque bras de flexion (22, 24) ayant sur au moins une partie de sa longueur une épaisseur (w) selon la direction de l'axe optique (XX') supérieure à sa largeur (h) dans un plan orthogonal à l'axe optique; **caractérisé en ce que** chaque ensemble haptique comprend
- un bras radial (16) s'étendant radialement par rapport à la partie optique (10) et ayant une première extrémité (16a) de raccordement à la partie optique et une deuxième extrémité (16b) raccordée à la première exremité desdits bras de flexion (22, 24).

2. Implant selon la revendication 1, **caractérisé en ce que** la face postérieure de chaque ensemble haptique comporte une portion allongée en saillie (28) s'étendant sur la deuxième extrémité du bras radial (16), sur les deux bras de flexion (22, 24) et sur une partie des éléments d'appui (18, 20), par quoi ladite portion allongée en saillie (28) rigidifie l'ensemble haptique dans le plan orthogonal à l'axe optique (XX').

3. implant selon l'une quelconque des revendications 1 et 2, **caractérisé en ce que** l'épaisseur (w) d'un bras de flexion (22, 24) est au moins égale à deux fois sa largeur (h).

4. Implant intraoculaire selon la revendication 2, **caractérisé en ce que** chaque élément d'appui (18, 20) comprend une face antérieure de forme convexe et une face postérieure présentant une forme concave entre ledit bord d'appui (18a, 20a) et ladite portion en saillie (28), la face convexe antérieure, le bord d'appui et la portion en saillie formant trois zones de contact avec la paroi interne de l'oeil.

5. Implant intraoculaire selon l'une quelconque des revendications 1 à 4, **caractérisé en ce que** chaque élément d'appui (18, 20) comprend en outre un bord de raccordement (18b, 20b) audit bras de flexion (22, 24) et un bord externe (18c, 20c) opposé au bord de raccordement, les raccordements entre, d'une part, le bord de contact et le bord de raccordement et, d'autre part, le bord de contact et le bord externe, n'étant pas arrondis.

6. Implant selon la revendication 5, **caractérisé en ce que** le bord externe (18c, 20c) de chaque élément d'appui est sensiblement rectiligne et fait un angle a avec l'axe commun (YY') aux bras radiaux (16) dont le sommet A est disposé, par rapport à la deuxième extrémité (16b) du bras radial, dans une direction opposée à celle du centre (0) de la partie optique (10).

7. Implant intraoculaire selon l'une quelconque des revendications 1 à 6, **caractérisé en ce que** la distance (H) entre le centre (0) de la partie optique (10) et la deuxième extrémité (16b) d'un bras radial est inférieure à la demi-longueur hors tout (H') de l'ensemble de l'implant.

## Claims

1. A one-piece intraocular implant made of a flexible material and comprising a substantially circular optical portion (10) having an optical axis XX' and a centre O, and a haptic portion constituted by two haptic assemblies (12, 14) that are substantially diametrically opposite each other, each haptic assembly comprises:
- two massive bearing elements (18, 20) that are substantially identical, each bearing element presenting a bearing edge (18a, 20a) for bearing against the inside wall of the eye; and
- two substantially identical flexing arms (22, 24), each flexing arm having a first end and a second end connected to a bearing element (18, 20) outside its bearing edge (18a, 20a), each flexing arm (22, 24) having, over at least a fraction of its length, a thickness (w) in the direction of the optical axis (XX') that is greater than its width (h) in a plane orthogonal to the optical axis; the implant being **characterised in that** each haptic assembly comprises a radial arm (16) extending radially relative to the optical portion (10) and having a connection first end (16a) connected to the optical portion, and a second end (16b) connected to the first ends of said flexing arms (22, 24).

2. An implant according to claim 1, **characterised in that** the posterior face of each haptic assembly includes an elongate projecting portion (28) standing proud on the second end of the radial arm (16), on the two flexing arms (22, 24), and on a fraction of the bearing elements (18, 20), whereby said elongate projecting portion (28) stiffens the haptic assembly in the plane orthogonal to the optical axis (XX').

3. An implant according to claim 1 or claim 2, **characterised in that** the thickness (w) of a flexing arm (22, 24) is not less than twice its width (h).

4. An intraocular implant according to claim 2, **characterised in that** each bearing element (18, 20) has an anterior face of convex shape and a posterior face presenting a concave shape between said bearing edge (18a, 20a) and said projecting portion (28), the anterior convex face, the bearing edge, and the projecting portion forming three contact zones with the inside wall of the eye.

5. An intraocular implant according to any one of claims 1 to 4, **characterised in that** each bearing element (18, 20) further comprises a connection edge (18b, 20b) connecting to said flexing arm (22, 24), and an outer edge (18c, 20c) opposite the connection edge, the connections firstly between the contact edge and the connection edge, and secondly between the contact edge and the outer edge, not being rounded.

6. An implant according to claim 5, **characterised in that** the outer edge (18c, 20c) of each bearing element is substantially rectilinear and makes an angle a with the axis (YY') that is common to the radial arms (16), with the vertex A of the angle being disposed relative to the second end (16b) of the radial arm in the opposite direction to the centre (O) of the optical portion (10).

7. An intraocular implant according to any one of claims 1 to 6, **characterised in that** the distance (H) between the centre (O) of the optical portion (10) and the second end (16b) of a radial arm is less than the overall half-length (H') of the implant as a whole.

## Patentansprüche

1. Intraokulares einstückiges Implantat, das aus einem flexiblen Material ausgeführt ist, umfassend einen optischen Teil (10), der im wesentlichen kreisförmig zu einer optischen Achse XX' und mit Zentrum 0 ist, und einen Haptikteil, der aus zwei Haptikkonstruktionen (12, 14) besteht, die sich im wesentlichen diametral gegenüberstehen, wobei jede Haptikkonstruktion umfaßt:
- zwei Halteelemente (18, 20), die massiv und im wesentlichen identisch sind, wobei jedes Halteelement einen Rand zur Abstützung (18a, 20a) auf der Innenwand des Auges aufweist, und
- zwei im wesentlichen identische Biegearme (22, 24), wobei jeder Biegearm ein erstes Ende und ein zweites Ende hat, die mit dem Halteelement (18, 20) außerhalb seines Randes zur Abstützung (18a, 20a) verbunden sind, wobei jeder Biegearm (22, 24) über wenigstens einen Teil seiner Länge eine Dicke (w) in der Richtung der optischen Achse (XX') besitzt, die größer ist als seine Breite (h) in einer zur optischen Achse orthogonalen Ebene,
**dadurch gekennzeichnet, daß** jede Haptikkonstruktion einen radialen Arm (16) umfaßt, der sich radial bezüglich des optischen Teils (10) erstreckt, und ein erstes Ende (16a) zur Verbindung mit dem optischen Teil und ein zweites Ende (16b) hat, das mit dem ersten Ende der Biegearme (22, 24) verbunden ist.

2. Implantat nach Anspruch 1, **dadurch gekennzeichnet, daß** die Hinterseite jeder Haptikkonstruktion einen langgestreckten, hervorstehenden Teil (28) hat, der sich über das zweite Ende des radialen Armes (16) erstreckt, über beide Biegearme (22, 24) und über einen Teil der Halteelemente (18, 20), wodurch der langgestreckte hervorstehende Teil (28) die Haptikkonstruktion in der zur optischen Achse (XX') orthogonalen Ebene versteift.

3. Implantat nach einem der Ansprüche 1 und 2, **dadurch gekennzeichnet, daß** die Dicke (w) eines Biegearms (22, 24) wenigstens gleich dem zweifachen seiner Breite (h) ist.

4. Intraokulares Implantat nach Anspruch 2, **dadurch gekennzeichnet, daß** jedes Halteelement (18, 20) eine Vorderseite mit konvexer Form und eine Hinterseite aufweist, die eine konkave Form, zwischen dem Rand zur Abstützung (18a, 20a) und dem hervorstehenden Teil (28) aufweist, wobei die konvexe Vorderseite, der Rand zur Abstützung und der hervorstehende Teil drei Kontaktzonen mit der Innenwand des Auges ausbilden.

5. Intraokulares Implantat nach einem der Ansprüche 1 bis 4, **dadurch gekennzeichnet, daß** jedes Halteelement (18, 20) außerdem einen Verbindungsrand (18b, 20b) mit dem Biegearm (22, 24) und einen Außenrand (18c, 20c) aufweist, der dem Verbindungsrand gegenübersteht, wobei die Verbindungen zwischen einerseits dem Kontaktrand und dem Verbindungsrand und andererseits dem Kontaktrand und dem Außenrand nicht abgerundet sind.

6. Implantat nach Anspruch 5, **dadurch gekennzeichnet, daß** der Außenrand (18c, 20c) jedes Halteelements im wesentlichen geradlinig ist und einen Winkel a mit der gemeinsamen Achse (YY') bei den Radialarmen (16) macht, dessen Spitze A im Verhältnis zu dem zweiten Ende (16b) des Radialarms in einer Richtung angeordnet ist, die jener des Zentrums (0) des optischen Teils (10) entgegengesetzt ist.

7. Intraokulares Implantat nach einem der Ansprüche 1 bis 6, **dadurch gekennzeichnet, daß** der Abstand (H) zwischen dem Zentrum (0) des optischen Teils (10) und dem zweiten Ende (16b) eines Radialarms kleiner als die halbe Länge über die gesamte Implantatkonstruktion (H') ist.
